(19) Europäisches Patentamt
European Patent Office
Office européen des brevets

(11) Veröffentlichungsnummer: **0 200 051**
**B1**

(12) **EUROPÄISCHE PATENTSCHRIFT**

(45) Veröffentlichungstag der Patentschrift: **12.09.90**

(51) Int. Cl.[5]: **C07C 257/02**, C07C 257/04, C07C 327/38

(21) Anmeldenummer: **86104953.4**

(22) Anmeldetag: **10.04.86**

(54) **Verfahren zur Herstellung von Imidaten.**

(30) Priorität: **22.04.85 DE 3514450**

(43) Veröffentlichungstag der Anmeldung: **10.12.86 Patentblatt 86/45**

(45) Bekanntmachung des Hinweises auf die Patenterteilung: **12.09.90 Patentblatt 90/37**

(84) Benannte Vertragsstaaten: **CH DE FR GB IT LI**

(56) Entgegenhaltungen:
**EP-A- 0 005 944**
**DE-A- 2 640 464**
**DE-B- 1 276 402**

**THE JOURNAL OF ORGANIC CHEMISTRY, Band 34, Nr. 3, März 1969, Seiten 627-629, Washington, DC, US; R.F. BORCH: "Nitrilium salts. A new method for the synthesis of secondary amines"**

(73) Patentinhaber: **HOECHST AKTIENGESELLSCHAFT, Postfach 80 03 20, D-6230 Frankfurt am Main 80(DE)**

(72) Erfinder: **Fuss, Andreas, Dr., Lindigstrasse 24, D-8757 Karlstein(DE)**
Erfinder: **Siegemund, Günter, Dr., Frankfurter Strasse 21, D-6238 Hofheim am Taunus(DE)**

**EP 0 200 051 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von Imidaten.

Es ist bekannt, Imidate aus Nitrilen und Alkoholen gemäß den Bedingungen der Pinner-Reaktion herzustellen. Hierbei werden ausschließlich Chlorwasserstoff, Bromwasserstoff, Jodwasserstoff oder Schwefelsäure als Reaktanden verwendet. Die Reaktionstemperatur ist wegen der thermischen Empfindlichkeit der zunächst entstehenden Imidatsalze auf niedere Reaktionstemperaturen (0 bis 5°C) limitiert (s. z.B. D.G. Nelson in S. Patai, The Chemistry of Amidines and Imidates, John Wiley & Sons, 1975, S. 385). Infolge der niederen Reaktionstemperaturen müssen lange Reaktionszeiten in Kauf genommen werden.

Ferner lassen sich mit dieser Pinner-Reaktion ortho-substituierte Benzonitrile wie beispielsweise o-Tolunitril, 2-Chlorbenzonitril, 2-Fluorbenzonitril oder 2,6-Difluorbenzonitril (vgl. H. Henecka und P. Kurtz in Houben-Weyl-Müller, Methoden der Organischen Chemie, Thieme Verlag, Stuttgart, Bd. 8 1952, S. 697) nicht einsetzen oder führen zu unbefriedigenden Ausbeuten. Außerdem wird auch bei sterisch gehinderten Nitrilen oder bei Nitrilen, die elektronenanziehende Substituenten enthalten wie Trichloracetonitril nur eine unvollständige Umsetzung oder überhaupt keine Reaktion beobachtet. Schwierigkeiten ergeben sich ferner auch bei der Verwendung von tertiären Alkoholen als Reaktionspartner.

Es wurde nun ein neues Verfahren zur Herstellung von Imidaten gefunden, das breiter anwendbar ist und anwendungstechnische Vorteile besitzt.

Gegenstand der Erfindung ist daher ein Verfahren zur Herstellung von Verbindungen der Formel I sowie ihrer Salze oder Säureadditionsverbindungen

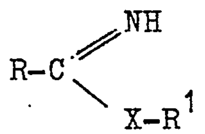

(I),

worin

R= Wasserstoff, $(C_1\text{-}C_{20})$Alkyl, halogeniertes $(C_1\text{-}C_{20})$Alkyl mit Ausnahme von $CF_3$, Naphthyl, Anthracenyl, Phenyl oder Phenyl, Anthracenyl, Naphthyl, die alle drei ein- oder mehrfach durch Halogen, Hydroxy, $(C_1\text{-}C_6)$Alkyl, $(C_1\text{-}C_6)$Alkylmercapto, $(C_1\text{-}C_6)$Alkoxy, $(C_1\text{-}C_6)$Alkylsulfonyl, $(C_1\text{-}C_6)$Alkylsulfinyl, $(C_1\text{-}C_6)$Alkylsulfonyloxy, wobei die vorgenannten alkylhaltigen Reste halogeniert sein können, Halogen, Nitro, $(C_1\text{-}C_6$-Alkoxy)carbonyl, Cyano, Phenyl, Phenylthio oder Phenoxy, wobei diese drei Reste ihrerseits durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy, Phenyl, Heteroaryl, Halogen substituiert sein können, substituiert sind; Heteroaryl, das durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy, Phenyl, Heteroaryl, Halogen substituiert sein kann; Phenoxy, Phenylthio, Phenylamino, wobei diese drei Reste durch $(C_1\text{-}C_6)$Alkyl, $(C_1\text{-}C_6)$-Halogenalkyl, $(C_1\text{-}C_6)$Alkoxy, $(C_1\text{-}C_6)$Halogenalkoxy, Phenyl, Heteroaryl, Halogen substituiert sein können; $(C_3\text{-}C_7)$Cycloalkyl, das halogeniert sein kann, Halogen, Hydroxy, $(C_1\text{-}C_{10})$Alkoxy, Mercapto, $(C_1\text{-}C_{10})$Alkylmercapto, Amino oder Mono- und Di$(C_1\text{-}C_{10}$alkyl)amino,

$R^1$= $(C_1\text{-}C_{12})$Alkyl, das ein- oder mehrfach durch Halogen, Nitro, $(C_1\text{-}C_4)$Alkoxy oder Carboxy substituiert sein kann, Phenyl, Naphthyl, die beide ein- oder mehrfach durch $(C_1\text{-}C_6)$Alkyl, $(C_1\text{-}C_6)$Halogenalkyl, $(C_1\text{-}C_6)$Alkoxy $(C_1\text{-}C_6)$Halogenalkoxy, Phenyl, Heteroaryl oder Halogen substituiert sein können; $(C_3\text{-}C_6)$Alkenyl, $(C_3\text{-}C_6)$Alkinyl, $(C_5\text{-}C_6)$Cycloalkyl oder Benzyl, das im Phenylring durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy, Phenyl, Heteroaryl, Halogen substituiert sein kann, oder Heteroaryl, das durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy oder Halogen substituiert sein kann, und

X= Sauerstoff oder Schwefel bedeutet,

dadurch gekennzeichnet, daß man ein Nitril der Formel II mit einer Verbindung der Formel III

$$R\text{-}C{\equiv}N \quad (II) \qquad\qquad R^1\text{-}XH \quad (III)$$

in Gegenwart von wasserfreier Flußsäure, Perfluoralkancarbonsäure, Perfluoralkansulfonsäure oder einem sulfonsäurehaltigen perfluorierten Ionenaustauscher umsetzt, und die erhaltenen Salze der Formel I gegebenenfalls unter Zusatz von Basen in die freien Imidate der Formel I überführt und diese gegebenenfalls anschließend in weitere Salze oder Säureadditionsverbindungen umwandelt.

Als Salze der Formel I kommen in Frage Salze mit anorganischen Säuren wie HCl, HBr, HJ, Schwefelsäure, Phosphorsäure oder organischen Carbonsäuren wie die obengenannten Perfluoralkancarbonsäuren, Perfluoralkansulfonsäuren, Tetrafluorborsäure, Hexafluorphosphorsäure, Pikrinsäure, $(C_1\text{-}C_6)$Alkansulfonsäuren;

Säureadditionsverbindungen werden insbesondere mit Flußsäure erhalten, wobei sich mehrere Moleküle HF an die Verbindung der Formel I addieren. Die Herstellung der Salze aus den Imidaten der Formel I erfolgt nach üblichen, dem Fachmann geläufigen Methoden.

Von den bei der Umsetzung der Verbindungen der Formel II mit den Verbindungen der Formel III benutzten Säuren finden insbesondere die Flußsäure und die Trifluormethansulfonsäure, besonders bevorzugt die Flußsäure, Anwendung. Die verwendete Flußsäure muß weitgehend wasserfrei sein. Der Wassergehalt darf nicht höher als 1 Gew.-% liegen. Als sulfonsäurehaltiger perfluorierter Ionenaustauscher kann im erfindungsgemäßen Verfahren das Handelsprodukt ®Nafion (Fa. DuPont, Wilmington) eingesetzt werden.

Als Heteroarylreste für R von Formel I kommen prinzipiell säurestabile Heteroarylreste infrage wie beispielsweise gegebenenfalls substituiertes Imidazolyl, Pyrazolyl, Triazolyl, Thiazolyl, Oxazolyl, Pyridyl, Dioxotetrahydropyridyl, Triazinyl, Pyrimidinyl, Pyrazinyl, Pyridazinyl, Chinolinyl, Benzthiazolyl(oxy), Chinoxalinyl(oxy), Thienyl, Furanyl, Pyrrolyl, Pyrrolidinyl. Unter Halogen-Resten sind insbesondere Fluor- oder Chlor-Reste zu verstehen.

Bevorzugt können nach dem erfindungsgemäßen Verfahren solche Verbindungen der Formel I hergestellt werden, worin

R= $(C_1$-$C_{20})$Alkyl, Phenyl, das durch $(C_1$-$C_6)$Alkyl, $(C_1$-$C_6)$Halogenalkyl, Halogen oder Hydroxy substituiert ist, $(C_3$-$C_7)$Cycloalkyl, das halogeniert sein kann, Pyridyl, Pyrrolyl, Furyl, Thienyl, Dioxotetrahydropyridyl, wobei diese Heterocyclen durch $(C_1$-$C_4)$Alkyl, $(C_1$-$C_6)$Halogenalkyl, $(C_1$-$C_6)$Alkoxy, $(C_1$-$C_6)$-Halogenalkoxy oder Halogen substituiert sein können.

$R^1$= $(C_1$-$C_{12})$Alkyl, das ein- oder mehrfach durch Halogen und/oder einfach durch Nitro oder Carboxy substituiert sein kann, Phenyl, das durch $(C_1$-$C_6)$Alkyl, $(C_1$-$C_6)$Halogenalkyl, $(C_1$-$C_6)$Alkoxy, $(C_1$-$C_6)$-Halogenalkoxy oder Halogen substituiert sein kann, $(C_5$-$C_6)$Cycloalkyl, Naphthyl, $(C_3$-$C_6)$Alkenyl, Pyridyl, Benzthiazolyl, die beide durch $(C_1$-$C_6)$Alkyl oder Halogen substituiert sein können, und

X= O oder S bedeuten.

Das erfindungsgemäße Verfahren läßt sich in einem breiten Temperaturbereich von -20° bis +200°C, vorzugsweise von 50° bis +150°C durchführen. Es kann drucklos oder im Autoklaven bei Drücken von 0 bis 100 bar gearbeitet werden.

Vorzugsweise kann bei der Umsetzung der Verbindung der Formel II und III eine Lewissäure hinzugesetzt werden. Als solche kommen beispielsweise infrage: Antimonpentachlorid, Antimontrifluorid, Phosphorpentachlorid, Phosphortrichlorid, Titantetrachlorid, Eisen(III)chlorid, Aluminiumtrichlorid, Zinkdichlorid, Bortrifluorid, Bortrichlorid, Zinn(IV)chlorid oder Kupfer(I)chlorid. Der Anteil an dem Katalysator bezogen auf 1 mol des eingesetzten Nitrils der Formel II kann im Bereich zwischen 0,001 bis 1,0 mol variieren.

Anstelle der obengenannten Lewissäurekatalysatoren kann auch ein Trichlormethylaromat wie Benzotrichlorid verwendet werden; dieser wird im Verhältnis von 0,01 bis 1,0 mol pro mol Nitril der Formel II verwendet. Es kann ferner als Katalysator eine Mineralsäure wie HCl, HBr, HJ in Mengen von 0,01 bis 1,0 mol pro mol Nitril zugegeben werden.

Ein Lösungsmittel ist für das erfindungsgemäße Verfahren nicht unbedingt notwendig. Prinzipiell können jedoch inerte Lösungsmittel wie Ether, Halogenkohlenwasserstoffe, Aromaten, Sulfoxide oder Sulfone oder überschüssige Säure verwendet werden.

Die Umsetzungen werden auf einfache Weise so durchgeführt, daß man die Verbindungen der Formeln II und III gegebenenfalls zusammen mit dem Katalysator und/oder dem Lösungsmittel in einem Stahl- oder Nickelautoklaven oder in einem Kessel aus geeignetem Werkstoff unter Inertgasatmosphere vorgelegt und die Säure hinzugepumpt. Nach der Reaktion entfernt man das Solvens und die Säure durch Destillation. Die zurückgewonnene Säure bzw. das Solvens können erneut in die Reaktion eingesetzt werden.

Die Imidate der Formel I können in Form ihrer Salze der bei der Umsetzung verwendeten Säuren isoliert werden oder mit Hilfe einer wäßrigen Base wie beispielsweise Kaliumcarbonat oder Kaliumhydroxid als freie Base erhalten werden. Die Imidate der Formel I können durch Destillation gereinigt werden. Vorteilhaft werden sie jedoch in Ether als Hydrochloride mittels Chlorwasserstoff oder als Tetrafluoroborate mittels $HBF_4$ ausgefällt.

Überraschenderweise verläuft das erfindungsgemäße Verfahren auch bei Verwendung von Nitrilen, die bei dem Verfahren des Standes der Technik nur unbefriedigende Umsetzungen ergeben, in hoher Ausbeute. Außerdem kann beim erfindungsgemäßen Verfahren die Raum-Zeit-Ausbeute stark erhöht werden. Das Verfahren eignet sich daher auch zur Herstellung von Imidaten im technischen Maßstab.

Die nach dem erfindungsgemäßen Verfahren hergestellten Imidate der Formel I oder deren Salze eignen sich als Vorprodukte für die Synthese von Pflanzenschutzmitteln (s. DE-OS 33 34 207) oder von Arzneimitteln (s. DE-OS 2 147 794).

Einige der nach dem Verfahren der Anmeldung hergestellten Imidate sind neu. Dies sind die Verbindungen der Formel I, bei denen $R^1$= einen Phenyl oder, wie oben angegeben, substituierten Phenylrest bedeutet und R, X die obengenannten Bedeutungen besitzen. Diese neuen Verbindungen der Formel I sind ebenfalls Gegenstand der vorliegenden Erfindung.

Die Erfindung wird durch nachstehende Beispiele erläutert.

Beispiel 1

1,39 g (10 mmol) 2,6-Difluorbenzonitril wurden in einem 10 ml VA-Autoklaven bei 0°C in 2 ml wasserfreier Flußsäure gelöst und 0,92 g (20 mmol) Ethanol zugesetzt. Man rührte 17 h bei 100°C, ließ Abkühlen

und dampfte die flüchtigen Bestandteile bei 50 - 60°C ab. Der Rückstand wurde in 20 ml $H_2O$ und 100 ml Ether gelöst und die wäßrige Phase mit 40 % wäßrigem KOH gegen Phenolphthalein neutralisiert.

Man extrahierte die wäßrige Phase mit 25 ml Ether und trocknete die vereinigten organischen Phasen über Natriumsulfat. Anschließend wurde das Produkt als Hydrochlorid durch Einleiten von trockenem Chlorwasserstoffgas gefällt. Man erhielt 1,22 g (55 %) (2,6-Difluorphenyl)-0-ethyl-imidat-hydrochlorid als farblosen kristallinen Feststoff, Schmp. 126 - 129°C.

Beispiel 2

1.03 g (10 mmol) Benzonitril, 2 ml wasserfreie Flußsäure, und 691 mg (15 mmol) Ethanol wurden, wie unter Beispiel 1 beschrieben, im VA-Autoklaven 17 h bei 100°C gerührt. Der abgekühlte Autklaveninhalt wurde auf 25 g Eis gegossen und das restliche Produkt mit $H_2O$ und Ether aus dem Autoklaven gespült. Man neutralisierte die wäßrige Phase mit wäßrigem Kaliumcarbonat und extrahierte das Produkt mit Ether.

Nach Trocknung der organischen Phasen über Natriumsulfat und Abdampfen der flüchtigen Bestandteile am Rotationsverdampfer blieb das Produkt Phenyl-0-ethylimidat als farbloses Öl zurück.

Beispiele 3 - 8

Allgemeine Vorschrift

In einem 10 ml VA-Autoklaven wurden (a) mol 2-Chlorbenzonitril und (b) mol Katalysator in 2 ml HF gelöst und mit (c) mol Ethanol versetzt. Die Reaktionsdauer, Temperatur sowie Molmengen lassen sich der nachfolgenden Tabelle 1 entnehmen. Die Aufarbeitung geschah, wie unter Beispiel 1 beschrieben. Es wurde jeweils 2-Chlorphenyl-0-ethyl-imidat-hydrochlorid, Schmp. 105 - 106°C (Zers.), als Produkt erhalten.

Tabelle 1

| Beispiel | a | b/Katalysator | c | Dauer [h] | Temp. [°C] | Ausbeute [g] |
|---|---|---|---|---|---|---|
| 3 | $10^{-2}$ | - | $1.10^{-2}$ | 17 | 110 | 1,02 |
| 4 | $10^{-2}$ | - | $1.10^{-2}$ | 1 | 110 | 0,22 |
| 5 | $10^{-2}$ | $10^{-3}/SbCl_5$ | $1.1\cdot10^{-2}$ | 17 | 70 | 1,43 |
| 6 | $10^{-2}$ | - | $1.10^{-2}$ | 5 | 140 | 1,25 |
| 7 | $10^{-2}$ | $10^{-3}/TiCl_4$ | $2\cdot10^{-2}$ | 17 | 70 | 1,50 |
| 8 | $10^{-2}$ | $10^{-3}/PCl_5$ | $2\cdot10^{-2}$ | 17 | 70 | 1,63 |

Beispiele 9 - 17

Allgemeine Vorschrift

In einem 10 ml VA-Autoklav wurden (a) mol der Verbindung der Formel II mit (b) mol der Verbindung der Formel III und (c) mol Phosphorpentachlorid gemäß nachfolgender Tabelle 2 in 2 ml HF 17 Stunden bei 70°C gerührt. Die Aufarbeitung erfolgte wie unter Beispiel 1 beschrieben.

Analog lassen sich auch die Verbindungen der Beispiele 18 - 46 herstellen.

Tabelle 2

| Beispiel | Verb.II | a | Verb.III | b | c | Ausbeute [g] | Schmp. [°C] |
|---|---|---|---|---|---|---|---|
| 9 | 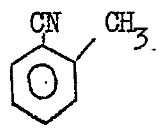 | $10^{-2}$ | EtOH | $2 \cdot 10^{-2}$ | $10^{-3}$ | 0,85 | 104–106 (Zers.) |
| 10 | 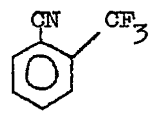 | $10^{-2}$ | EtOH | $2 \cdot 10^{-2}$ | $10^{-3}$ | 0,47 | 103–105 (Zers.) |
| 11 | 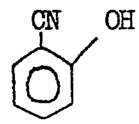 | $10^{-2}$ | EtOH | $2 \cdot 10^{-2}$ | $10^{-3}$ | 1,50 | 137 (Zers.) |
| 12 | t-Bu-CN | $10^{-2}$ | EtOH | $2 \cdot 10^{-2}$ | $10^{-3}$ | 0,71 | 133–135 (Zers.) |
| 13 | 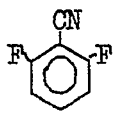 | $10^{-2}$ | $C_6H_5OH$ | $2 \cdot 10^{-2}$ | $10^{-3}$ | 0,64 | 106–108 (Zers.) |
| 14 | 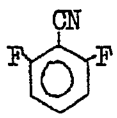 | $10^{-2}$ | $C_6H_5SH$ | $2 \cdot 10^{-2}$ | $10^{-3}$ | 0,40 | 220–232 |
| 15 1) | 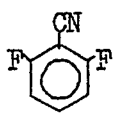 | $10^{-2}$ | EtSH | $2 \cdot 10^{-2}$ | $10^{-3}$ | 1,13 | 254–261 |
| 16 | 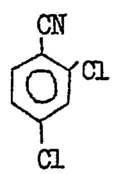 | $10^{-2}$ | EtOH | $2 \cdot 10^{-2}$ | $10^{-3}$ | 0,78 | 186–188 |
| 17 | 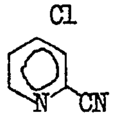 | $10^{-2}$ | EtOH | $2 \cdot 10^{-2}$ | $10^{-3}$ | 1,16 | 145–147 |
| 18 | $C_3H_7$-CN | | EtOH | | | | |
| 19 | $CH_2$=CH-CN | | EtOH | | | | |
| 20 | 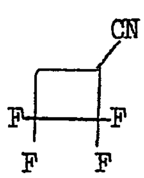 | | EtOH | | | | |

Tabelle 2 – Fortsetzung

| Beispiel | Verb.II | Verb.III |
|---|---|---|
| 21 | $C_{16}H_{33}-CN$ | EtOH |
| 22 | $C_6F_5-CN$ | EtOH |
| 23 | 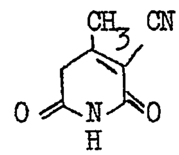 | EtOH |
| 24 | HCN | EtOH |
| 25 | 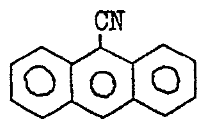 | EtOH |
| 26 | 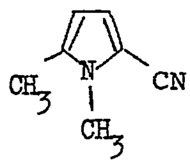 | EtOH |
| 27 | 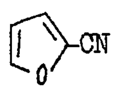 | EtOH |
| 28 | 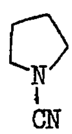 | EtOH |
| 29 | 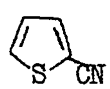 | EtOH |
| 30 | 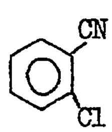 | $CH_2=CH-CH_2OH$ |
| 31 | " | $C_{11}H_{23}OH$ |
| 32 | " | 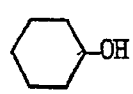 |
| 33 | " | $CF_3CH_2OH$ |

Tabelle 2 - Fortsetzung

| Beispiel | Verb.II | Verb.III |
|---|---|---|
| 34 | 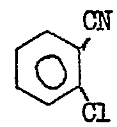 | 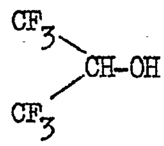 |
| 35 | " | $C_8F_{17}CH_2CH_2OH$ |
| 36 | 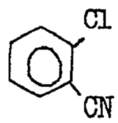 | $O_2N-CH_2CH_2OH$ |
| 37 | " | $Cl_3CCH_2OH$ |
| 38 | " | $CH_3O-CH_2CH_2OH$ |
| 39 | " | $HO_2C-CH_2OH$ |
| 40 | " | 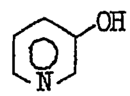 |
| 41 | " | 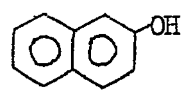 |
| 42 | " | $C_6F_5-OH$ |
| 43 | " | $CH_2=CH-CH_2-SH$ |
| 44 | " | $HO_2C-CH_2SH$ |
| 45 | " | 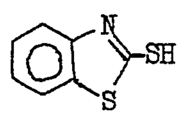 |
| 46 | " | 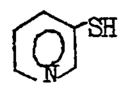 |

1) als Tetrafluoroborat gefällt

## Patentansprüche

1. Verfahren zur Herstellung von Verbindungen der Formel I und ihrer Salze oder Säureadditionsverbindungen

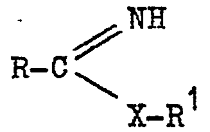

(I),

worin

R= Wasserstoff, $(C_1\text{-}C_{20})$Alkyl, halogeniertes $(C_1\text{-}C_{20})$Alkyl mit Ausnahme von $CF_3$, Naphthyl, Anthracenyl, Phenyl oder Phenyl, Anthracenyl, Naphthyl, die alle drei ein- oder mehrfach durch Halogen, Hydroxy, $(C_1\text{-}C_6)$-Alkyl, $(C_1\text{-}C_6)$Alkylmercapto, $(C_1\text{-}C_6)$Alkoxy, $(C_1\text{-}C_6)$Alkylsulfonyl, $(C_1\text{-}C_6)$Alkylsulfinyl, $(C_1\text{-}C_6)$Alkylsulfonyloxy, wobei die vorgenannten alkylhaltigen Reste halogeniert sein können, Halogen, Nitro, $(C_1\text{-}C_6$-Alkoxy)carbonyl, Cyano, Phenyl, Phenylthio oder Phenoxy, wobei diese drei Reste ihrerseits durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy, Phenyl, Heteroaryl, Halogen substituiert sein können, substituiert sind; Heteroaryl, das durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy, Phenyl, Heteroaryl, Halogen substituiert sein kann; Phenoxy, Phenylthio, Phenylamino, wobei diese drei Reste durch $(C_1\text{-}C_6)$Alkyl, $(C_1\text{-}C_6)$-Halogenalkyl, $(C_1\text{-}C_6)$Alkoxy, $(C_1\text{-}C_6)$Halogenalkoxy, Phenyl, Heteroaryl, Halogen substituiert sein können; $(C_3\text{-}C_7)$Cycloalkyl, das halogeniert sein kann,

Halogen, Hydroxy $(C_1\text{-}C_{10})$ Alkoxy, Mercapto, $(C_1\text{-}C_{10})$Alkylmercapto, Amino oder Mono- und Di$(C_1\text{-}C_{10}$alkyl)amino

$R^1$= $(C_1\text{-}C_{12})$Alkyl, das ein- oder mehrfach durch Halogen, Nitro, $(C_1\text{-}C_4)$Alkoxy oder Carboxy substituiert sein kann, Phenyl, Naphthyl, die beide ein- oder mehrfach durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy, Phenyl, Heteroaryl oder Halogen substituiert sein können, $(C_3\text{-}C_6)$Alkenyl, $(C_3\text{-}C_6)$Alkinyl, $(C_5\text{-}C_6)$Cycloalkyl oder Benzyl, das im Phenylring durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy, Phenyl, Heteroaryl, Halogen substituiert sein kann,

oder Heteroaryl, das durch $(C_1\text{-}C_6)$Alkyl, $(C_1\text{-}C_6)$Halogenalkyl, $(C_1\text{-}C_6)$Alkoxy, $(C_1\text{-}C_6)$Halogenalkoxy oder Halogen substituiert sein kann, und

X= Sauerstoff oder Schwefel bedeutet, dadurch gekennzeichnet, daß man ein Nitril der Formel II mit einer Verbindung der Formel III

$R\text{-}C{\equiv}N$ (II) $R^1\text{-}XH$ (III)

in Gegenwart von wasserfreier Flußsäure, Perfluoralkancarbonsäure, Perfluoralkansulfonsäure oder einen sulfonsäurehaltigen perfluorierten Ionenaustauscher umsetzt und die erhaltenen Salze der Formel I gegebenenfalls unter Zusatz von Basen in die freien Imidate der Formel I überführt und diese gegebenenfalls anschließend in weitere Salze oder Säureadditionsverbindungen umwandelt.

2. Verfahren gemäß Anspruch 1, dadurch gekennzeichnet, daß man wasserfreie Flußsäure oder Trifluormethansulfonsäure einsetzt.

3. Verfahren gemäß Ansprüchen 1 oder 2, dadurch gekennzeichnet, daß man wasserfreie Flußsäure einsetzt.

4. Verfahren gemäß Ansprüchen 1 bis 3, dadurch gekennzeichnet, daß man zusätzlich eine Lewissäure als Katalysator einsetzt.

5. Verfahren gemäß Ansprüchen 1 bis 4, dadurch gekennzeichnet, daß man zusätzlich einen Katalysator aus der Gruppe Antimonpentachlorid, Antimontrifluorid, Phosphorpentachlorid, Phosphortrichlorid, Titantetrachlorid, Eisen(III)-chlorid, Aluminiumtrichlorid, Zinkdichlorid, Bortrifluorid, Bortrichlorid, Kupfer(I) oder Zinn(IV)chlorid einsetzt.

6. Verfahren gemäß Ansprüchen 1 bis 3, dadurch gekennzeichnet, daß man zusätzlich ein Trichlormethylaromat oder geringe Mengen an einer Mineralsäure als Katalysator zusetzt.

7. Verfahren gemäß Ansprüchen 1 bis 6, dadurch gekennzeichnet, daß es im Temperaturbereich von –20° bis +200°C durchgeführt wird.

8. Verfahren gemäß Ansprüchen 1 bis 7, dadurch gekennzeichnet, daß es im Temperaturbereich von 50° bis 150°C durchgeführt wird.

9. Verfahren gemäß Ansprüchen 4 und 5, dadurch gekennzeichnet, daß der Lewissäure-Katalysator in einer Menge von 0,001 bis 1 mol pro mol Nitril der Formel II eingesetzt wird.

10. Verfahren gemäß Anspruch 6, dadurch gekennzeichnet, daß der Trichlormethylaromat in einer Menge von 0,01 bis 1 mol pro mol Nitril der Formel II eingesetzt wird.

11. Verfahren gemäß einem der Ansprüche 1 bis 10, dadurch gekennzeichnet, daß in Formel I

R= $(C_1\text{-}C_{20})$Alkyl, Phenyl, das durch $(C_1\text{-}C_6)$Alkyl, $(C_1\text{-}C_6)$Halogenalkyl, Halogen oder Hydroxy substituiert ist, $(C_3\text{-}C_7)$Cycloalkyl, das halogeniert sein kann, Pyridyl, Pyrrolyl, Furyl, Thienyl, Dioxotetrahydropyridyl, wobei diese Heterocyclen durch ($C_1$-$C_4$)Alkyl, ($C_1$-$C_6$)Halogenalkyl, ($C_1$-$C_6$)Alkoxy, ($C_1$-$C_6$)-Halogenalkoxy oder Halogen substituiert sein können,
$R^1$= ($C_1$-$C_{12}$)Alkyl, das ein- oder mehrfach durch Halogen und/oder einfach durch Nitro oder Carboxy substituiert sein kann, Phenyl, das durch ($C_1$-$C_6$)Alkyl, ($C_1$-$C_6$)Halogenalkyl, ($C_1$-$C_6$)Alkoxy, ($C_1$-$C_6$)-Halogenalkoxy oder Halogen substituiert sein kann, ($C_5$-$C_6$)Cycloalkyl, Naphthyl, ($C_3$-$C_6$)Alkenyl, Pyridyl, Benzthiazolyl, die beide durch ($C_1$-$C_6$)Alkyl oder Halogen substituiert sein können, und
X= O oder S bedeuten.

## Claims

1. A process for the preparation of compounds of the formula I and salts or acid addition compounds thereof

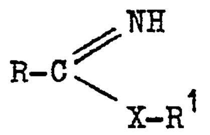

(I)

in which
R denotes hydrogen, ($C_1$–$C_{20}$)-alkyl, halogenated ($C_1$–$C_{20}$)-alkyl with the exception of $CF_3$, naphthyl, anthracenyl or phenyl or phenyl, anthracenyl or naphthyl all three of which are monosubstituted or polysubstituted by halogen, hydroxyl, ($C_1$–$C_6$)-alkyl, ($C_1$–$C_6$)-alkylmercapto, ($C_1$–$C_6$)-alkoxy, ($C_1$–$C_6$)-alkylsulfonyl, ($C_1$–$C_6$)-alkylsulfinyl or ($C_1$–$C_6$)-alkylsulfonyloxy, it being possible for the abovementioned alkyl-containing radicals to be halogenated, halogen, nitro ($C_1$–$C_6$-alkoxy)-carbonyl, cyano, phenyl, phenylthio or phenoxy, it being possible for these three radicals to be substituted in turn by ($C_1$–$C_6$)-alkyl, ($C_1$–$C_6$)-halogenoalkyl, ($C_1$–$C_6$)-alkoxy, ($C_1$–$C_6$)-halogenoalkoxy, phenyl, heteroaryl or halogen; heteroaryl which can be substituted by ($C_1$–$C_6$)-alkyl, ($C_1$–$C_6$)-halogenoalkyl, ($C_1$–$C_6$)-alkoxy, ($C_1$–$C_6$)-halogenoalkoxy, phenyl, heteroaryl or halogen; phenoxy, phenylthio or phenylamino, it being possible for these three radicals to be substituted by ($C_1$–$C_6$)-alkyl, ($C_1$–$C_6$)-halogenoalkyl, ($C_1$–$C_6$)-alkoxy, ($C_1$–$C_6$)-halogenoalkoxy, phenyl, heteroaryl or halogen; or ($C_3$–$C_7$)-cycloalkyl which can be halogenated, halogen, hydroxyl, ($C_1$–$C_{10}$)-alkoxy, mercapto, ($C_1$–$C_{10}$)-alkylmercapto, amino, mono-($C_1$–$C_{10}$-alkyl)-amino and di-($C_1$–$C_{10}$-alkyl)-amino,
$R^1$ denotes ($C_1$–$C_{12}$)-alkyl which can be monosubstituted or polysubstituted by halogen, nitro, ($C_1$–$C_4$)-alkoxy or carboxyl, phenyl or naphthyl both of which can be monosubstituted or polysubstituted by ($C_1$–$C_6$)-alkyl, ($C_1$–$C_6$)-halogenoalkyl, ($C_1$–$C_6$)-alkoxy, ($C_1$–$C_6$)-halogenoalkoxy, phenyl, heteroaryl or halogen, ($C_3$–$C_6$)-alkenyl, ($C_3$–$C_6$)-alkinyl, ($C_5$–$C_6$)-cycloalkyl or benzyl which can be substituted in the phenyl ring by ($C_1$–$C_6$)-alkyl, ($C_1$–$C_6$)-halogenoalkyl, ($C_1$–$C_6$)-alkoxy, ($C_1$–$C_6$)-halogenoalkoxy, phenyl heteroaryl or halogen, or heteroaryl which can be substituted by ($C_1$–$C_6$)-alkyl, ($C_1$–$C_6$)-halogenoalkyl, $C_1$–$C_6$)-alkoxy, ($C_1$–$C_6$)-halogenoalkoxy or halogen, and X denotes oxygen or sulfur, which comprises reacting a nitrile of the formula II with a compound of the formula III

$$R\text{–}C{\equiv}N \qquad (II) \qquad\qquad R^1\text{–}XH \qquad (III)$$

in the presence of anhydrous hydrofluoric acid, a perfluoroalkanecarboxylic acid, a perfluoroalkanesulfonic acid or a perfluorinated ion exchanger containing sulfonic acids, and converting the resulting salts of formula I, if appropriate with the addition of bases, into the free imidates of formula I and, if appropriate, subsequently converting the latter into further salts or acid addition compounds.

2. The process as claimed in claim 1, wherein anhydrous hydrofluoric acid or trifluoromethanesulfonic acid is employed.

3. The process as claimed in claims 1 or 2, wherein anhydrous hydrofluoric acid is employed.

4. The process as claimed in any of claims 1 to 3, wherein a Lewis acid is employed additionally as a catalyst.

5. The process as claimed in any of claims 1 to 4, wherein a catalyst belonging to the group comprising antimony pentachloride, antimony trifluoride, titaniumtetrachloride, iron (III) chloride, aluminum trichloride, zinc dichloride, boron trifluoride, boron trichloride, copper(I) chloride or thin (IV) chloride is employed additionally.

6. The process as claimed in any of claims 1 to 3, wherein a trichloromethyl aromatic compound or small amounts of a mineral acid are employed additionally as a catalyst.

7. The process as claimed in any of claims 1 to 6, wherein the process is carried out within the temperature range from –20°C to +200°C.

8. The process as claimed in any of claims 1 to 7, wherein the process is carried out within the temperature range from 50° to 150°C.

9. The process as claimed in claims 4 to 5, wherein the Lewis acid catalyst is employed in an amount of 0.001 to 1 mole per mole of nitrile of the formula II.

10. The process as claimed in claim 6, wherein the trichloromethyl aromatic compound is employed in an amount of 0.01 to 1 mole per mole of nitrile of the formula II.

11. The process as claimed in any of claims 1 to 10, wherein, in formula I,
R denotes ($C_1$–$C_{10}$)-alkyl, phenyl which is substituted by ($C_1$–$C_6$)-alkyl, ($C_1$–$C_6$)-halogenoalkyl, halogen or hydroxyl, ($C_3$–$C_7$)-cycloalkyl which can be halogenated, or pyridyl, pyrrolyl, furyl, thienyl or dioxotetrahydropyridyl, it being possible for these heterocyclic radicals to be substituted by ($C_1$–$C_4$)-alkyl, ($C_1$–$C_6$)-halogenoalkyl, ($C_1$–$C_6$)-alkoxy, ($C_1$–$C_6$)-halogenoalkoxy or halogen,
$R^1$ denotes ($C_1$–$C_{12}$)-alkyl which can be monosubstituted or polysubstituted by halogen and/or monosubstituted by nitro or carboxyl, phenyl which can be substituted by ($C_1$–$C_6$)-alkyl, ($C_1$–$C_6$)-halogenoalkyl, ($C_1$–$C_6$)-alkoxy, ($C_1$–$C_6$)-halogenoalkoxy or halogen, ($C_5$–$C_6$)-cycloalkyl, naphthyl or ($C_3$–$C_6$)-alkenyl or pyridyl or benzothiazolyl both of which can be substituted by ($C_1$–$C_6$)-alkyl or halogen, and
X denotes O or S.

## Revendications

1. Procédé pour préparer des composés de formule I, et leur sels ou composés d'addition d'acide,

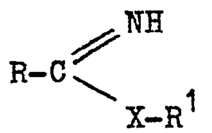

(I),

dans laquelle
R représente un atome d'hydrogène, un groupe alkyle en $C_1$ à $C_{20}$, un groupe alkyle en $C_1$ à $C_{20}$, halogéné mais à l'exception de $CF_3$, un groupe naphtyle, anthracényle, phényle, ou bien un groupe phényle, anthracényle ou naphtyle (ces trois groupes étant chacun substitués une ou plusieurs fois par de l'halogène, par un groupe hydroxy, alkyle en $C_1$ à $C_6$, alkylmercapto en $C_1$ à $C_6$, alcoxy en $C_1$ à $C_6$, alkyl (en $C_1$ à $C_6$) sulfonyle, alkyl (en $C_1$ à $C_6$) sulfinyle, alkyl (en $C_1$ à $C_6$) sulfonyloxy, les restes précités contenant un groupe alkyle pouvant être halogéné, un atome d'halogène, un groupe nitro, alcoxy en $C_1$ à $C_6$ carbonyle, cyano, phényle, phénylthio ou phénoxy, ces trois restes pouvant être pour leur part substitués par un groupe alkyle en $C_1$ à $C_6$, halogénoalkyle en $C_1$ à $C_6$, alcoxy en $C_1$ à $C_6$, halogéno-alcoxy en $C_1$ à $C_6$, phényle, hétéro-aryle, par de l'halogène); un groupe hétéro-aryle, qui peut être substitué par un groupe alkyle en $C_1$ à $C_6$, halogéno-alkyle en $C_1$ à $C_6$, alcoxy en $C_1$ à $C_6$, halogéno-alcoxy en $C_1$ à $C_6$, phényle, hétéro-aryle, par de l'halogène; un groupe phénoxy, phénylthio, phénylamino (ces trois restes pouvant être substitués par un groupe alkyle en $C_1$ à $C_6$, halogéno-alkyle en $C_1$ à $C_6$, alcoxy en $C_1$ à $C_6$, halogéno-alcoxy en $C_1$ à $C_6$, phényle, hétéro-aryle, par de l'halogène); un groupe cycloalkyle en $C_3$ à $C_7$, qui peut être halogéné, de l'halogène, un groupe hydroxy, un groupe alcoxy en $C_1$ à $C_{10}$, mercapto, alkyle (en $C_1$ à $C_{10}$) mercapto, amino ou monoalkylamino ou dialkylamino dont le groupe alkyle comporte 1 ou 10 atomes de carbone,
$R^1$ représente un groupe alkyle en $C_1$ à $C_{12}$ (qui peut être substitué une ou plusieurs fois par de l'halogène, par un groupe nitro, par un groupe alcoxy en $C_1$ à $C_4$ ou par un groupe carboxy), un groupe phényle, naphtyle (ces deux groupes pouvant être substitués une ou plusieurs fois par un groupe alkyle en $C_1$ à $C_6$, halogéno-alkyle en $C_1$ à $C_6$, alcoxy en $C_1$ à $C_6$, halogéno-alkoxy en $C_1$ à $C_6$, phényle, hétéro-aryle ou halogéno); un groupe alcényle en $C_3$ à $C_6$, alcynyle en $C_3$ à $C_6$, cycloalkyle en $C_5$ à $C_6$ ou benzyle (pouvant être substitué par le noyau phényle par un groupe alkyle en $C_1$ à $C_6$, halogénoalkyle en $C_1$ à $C_6$, alcoxy en $C_1$ à $C_6$, halogénoalcoxy en $C_1$ à $C_6$, phényle, hétéroaryle, par de l'halogène), ou un groupe hétéroaryle (pouvant être substitué par un groupe alkyle en $C_1$ à $C_6$, halogénoalkyle en $C_1$ à $C_6$, alcoxy en $C_1$ à $C_6$, halogéno-alcoxy en $C_1$ à $C_6$ ou par de l'halogène), et
X représente un atome d'oxygène ou de soufre,
procédé caractérisé en ce qu'on fait réagir un nitrile de formule II avec un composé de formule III

$R{-}C{\equiv}N$ (II) $R^1{-}XH$ (III)

en présence d'acide fluorhydrique anhydre, d'un acide perfluoro-alcane-carboxylique, d'un acide perfluoro-alcane-sulfonique ou d'un échangeur d'ions perfluoré et contenant de l'acide sulfonique, et l'on transforme éventuellement les sels ainsi obtenus, de formule I, par addition de base en les imidates libres de formule I et on transforme ceux-ci éventuellement ensuite en d'autres sels ou en des composés d'addition d'acides.

2. Procédé selon la revendication 1, caractérisé en ce qu'on utilise de l'acide fluorhydrique anhydre ou de l'acide trifluorométhane sulfonique.

3. Procédé selon les revendications 1 ou 2, caractérisé en ce qu'on utilise de l'acide fluorhydrique anhydre.

4. Procédé selon les revendications 1 à 3, caractérisé en ce qu'on utilise en outre comme catalyseur un acide de Lewis.

5. Procédé selon les revendications 1 à 4, caractérisé en ce qu'on utilise en outre un catalyseur choisi parmi le pentachlorure d'antimoine, le trifluorure d'antimoine, le pentachlorure de phosphore, le trichlorure de phosphore, le tétrachlorure de titane, le chlorure de fer (III), le trichlorure d'aluminium, le dichlorure de zinc, le trifluorure de bore, le trichlorure de bore, le chlorure de cuivre (I) ou le chlorure d'étain (IV).

6. Procédé selon les revendications 1 à 3, caractérisé en ce qu'on ajoute également, comme catalyseur, un composé trichlorométhyl aromatique de faible quantité d'un acide minéral.

7. Procédé selon les revendications 1 à 6, caractérisé en ce qu'il est conduit dans une gamme de la température allant de −20°C à +200°C.

8. Procédé selon les revendications 1 à 7, caractérisé en ce qu'il est conduit dans une gamme de la température allant de +50° à +150°C.

9. Procédé selon les revendications 4 et 5, caractérisé en ce qu'on utilise comme catalyseur l'acide de Lewis en une quantité de 0,001 à 1 mole par mole de nitrile de formule II.

10. Procédé selon la revendication 6, caractérisé en ce qu'on utilise le composé trichlorométhylaromatique en une quantité de 0,01 à 1 mole par mole de nitrile de formule II.

11. Procédé selon l'une des revendications 1 à 10, caractérisé en ce que, dans la formule I,
R représente un groupe alkyle en $C_1$ à $C_{20}$, phényle (qui est substitué par un groupe alkyle en $C_1$ à $C_6$, halogéno-alkyle en $C_1$ à $C_6$, halogéno ou hydroxy), un groupe cycloalkyle en $C_3$ à $C_7$, qui peut être halogène, un groupe pyridyle, pyrrolyle, furyle, thiényle, dioxotétrahydropyridyle, ces hétérocycles pouvant être substitués par un groupe alkyle en $C_1$ à $C_4$, halogénoalkyle en $C_1$ à $C_6$, alcoxy en $C_1$ à $C_6$, halogéno-alcoxy en $C_1$ à $C_6$ ou par de l'halogène;
$R^1$ représente un groupe alkyle en $C_1$ à $C_{12}$ (qui peut être substitué une ou plusieurs fois par de l'halogène et/ou une fois par un groupe nitro ou carboxy), un groupe phényle (qui peut être substitué par un groupe alkyle en $C_1$ à $C_6$, halogénoalkyle en $C_1$ à $C_6$, alcoxy en $C_1$ à $C_6$, halogénoalcoxy en $C_1$ à $C_6$ ou par de l'halogène), un groupe cycloalkyle en $C_5$ à $C_6$, naphtyle, alcényle en $C_3$ à $C_6$, pyridyle, benzothiazolyle, ces deux groupes pouvant être substitués par un groupe alkyle en $C_1$ à $C_6$ ou par de l'halogène, et
X représente O ou S.